# EUROPÄISCHE PATENTSCHRIFT

(11) **EP 3 142 582 B1**
(45) Veröffentlichungstag und Bekanntmachung des Hinweises auf die Patenterteilung: **09.02.2022**
(21) Anmeldenummer: 15726027.4
(22) Anmeldetag: 15.05.2015
(51) Int. Cl.: A61B 18/12

(54) **HOCHFREQUENZ-CHIRURGIEGERÄT**
HIGH-FREQUENCY SURGICAL APPLIANCE
APPAREIL CHIRURGICAL À HAUTE FRÉQUENCE

(30) Priorität: 15.05.2014 DE 102014209264
(43) Veröffentlichungstag der Anmeldung: 22.03.2017
(73) Patentinhaber: Olympus Winter & Ibe GmbH, 22045 Hamburg (DE)
(72) Erfinder: FISCHER, Uwe, 10439 Berlin (DE); KIRFE, Tino, 04617 Lödla (DE); SCHIDDEL, Stefan, 14532 Stahnsdorf (DE); KERSTEN, Lutz, 12163 Berlin (DE); BREITSPRECHER, Frank, 12527 Berlin (DE)
(74) Vertreter: Eisenführ Speiser
(86) Internationale Anmeldenummer: PCT/EP2015/060738
(87) Internationale Veröffentlichungsnummer: WO 2015/173382

(56) Entgegenhaltungen:
- US-A- 6 142 992
- US-A1- 2009 112 204
- US-A1- 2012 215 213

## Beschreibung

Die Erfindung betrifft ein Hochfrequenz-Chirurgiegerät mit einer Hochspannungs-Stromversorgungseinheit und einem Hochfrequenzgenerator, der im Betrieb von der Hochspannungs-Stromversorgungseinheit gespeist wird und einen hochfrequenten Wechselstrom erzeugt und an eine Last, beispielsweise ein Chirurgie-Instrument, abgibt. Derartige Hochfrequenz-Chirurgiegeräte sind aus dem Stand der Technik bekannt, beispielsweise aus US 6,142,992, EP 0 430 929 A2 oder WO 2006/050888 A1. Derartige Hochfrequenz-Chirurgiegeräte dienen dazu, ein entsprechendes chirurgisches Instrument zum Zwecke der Koagulation und des Schneidens von Gewebe mit Energie zu versorgen. Ein Problem stellen dabei regelmäßig Stromspitzen, beispielsweise infolge von Spannungsüberschlägen, die beispielsweise zu Lichtbogen führen können, dar. Dementsprechend werden in den genannten Veröffentlichungen Steuerungen beschrieben, die derartige Spannungsüberschläge verhindern bzw. beenden sollen. Auch in US 2012/215213 A1 und US 6,142,992 A sind derartige Steuerungen beschrieben.Es besteht jedoch nach wie vor Bedarf an einer schnellen und zuverlässigen Steuerung.

Der Schutzumfang der Erfindung wird im unabhängigen Anspruch 1 offenbart und bevorzugte Ausführungsformen werden in den abhängigen Ansprüchen beschrieben. Erfindungsgemäß wird hierfür ein Hochfrequenz-Chirurgiegerät der eingangs genannten Art vorgeschlagen, das eine Spitzenwertdetektions- und Steuereinheit aufweist, die ihrerseits einen Strom- oder Spannungssensor aufweist, der zum Erfassen der Stromstärke oder der Spannung eines im Betrieb an die Last abgegebenen hochfrequenten Wechselstroms ausgebildet ist und einen Effektivwertbildner aufweist oder mit diesem verbunden ist, wobei der Effektivwertbildner mit dem Strom- oder Spannungssensor verbunden und ausgebildet ist, einen Effektivwert eines von dem Strom- oder Spannungssensor erfassten hochfrequenten Wechselstroms zu bilden. Erfindungsgemäß ist die Spitzenwertdetektions- und Steuereinheit ausgebildet, dann, wenn im eingeschwungenen Zustand des HF-Hochspannungskreises ein Betrag eines momentanen Spitzenwertes eines einen Momentanwert des den von dem Strom- oder Spannungssensor im Betrieb erfassten hochfrequenten Wechselstroms repräsentierenden Signals einen Betrag eines vom Effektivwert des von dem Strom- oder Spannungssensor im Betrieb erfassten hochfrequenten Wechselstroms abgeleiteten Wertes überschreitet, den Hochfrequenzgenerator zu deaktivieren. Im Betrieb wird der HF-Hochspannungskreis von dem Hochfrequenzgenerator und dem daran angeschlossenen chirurgischen Instrument gebildet. Der Strom- oder Spannungssensor erfasst kontinuierlich den Momentanwert des im Betrieb in den HF-Hochspannungskreis fließenden Stroms oder der Spannung.

"Überschreiten" ist hier immer auf die Beträge des den jeweiligen Momentanwert repräsentierenden Signals und des von dem Effektivwert abgeleiteten Wertes bezogen.

Das einen Momentanwert des von dem Strom- oder Spannungssensor im Betrieb erfassten hochfrequenten Wechselstroms repräsentierende Signal kann gedämpft sein. Dann kann der vom Effektivwert des von dem Strom- oder Spannungssensor im Betrieb erfassten hochfrequenten Wechselstroms abgeleitete Wert unmittelbar dem Effektivwert entsprechen. Wichtig ist nur, dass die Maximal-Amplitude des den von dem Strom- oder Spannungssensor im Betrieb erfassten hochfrequenten Wechselstroms repräsentierenden Signals (also das den jeweiligen Momentanwert repräsentierende Signal) im stabilen, eingeschwungenen Zustand des HF-Hochspannungskreises kleiner ist, als der von dem Effektivwert des von dem Strom- oder Spannungssensor im Betrieb erfassten hochfrequenten Wechselstroms abgeleitete Wert, so dass das den jeweiligen Momentanwert repräsentierende Signal den vom Effektivwert des von dem Strom- oder Spannungssensor im Betrieb erfassten hochfrequenten Wechselstroms abgeleiteten Wert nur im Falle eines plötzlichen Anstiegs der Maximal-Amplitude des Momentanwertes überschreitet. Die Erfindung macht sich die Tatsache zu Nutze, dass ein Effektivwert erst mit einer gewissen Verzögerung einem Anstieg der Maximal-Amplitude der zugehörigen Momentanwerte folgt.

Zum Vergleich des jeweiligen Momentanwertes des von dem Strom- oder Spannungssensor erfassten Stroms mit dem von dem Effektivwert dieses Stroms abgeleiteten Wert weist die Spitzenwertdetektions- und Steuereinheit vorzugsweise einen Komparator auf, der als Hardware-Komponente, d.h. als analoge Schaltung, realisiert ist und daher sehr schnell reagieren kann.

Die Erfindung schließt die Erkenntnis ein, dass sich der Effektivwert des Stroms auch im Falle von Stromspitzen, wie sie beispielsweise bei einem Spannungsüberschlag (Lichtbogen) auftreten, langsamer steigt als der jeweilige Momentanwert. Dementsprechend steigt auch ein von dem Effektivwert abgeleiteter Wert vergleichsweise langsam. Wenn der von dem Effektivwert abgeleitete Wert beispielsweise der jeweilige Effektivwert plus ein fester Zuschlagswert ist, so dass das Deaktivieren des Hochfrequenzgenerators dann erfolgt, wenn der Momentanwert des hochfrequenten Wechselstroms den Effektivwert plus dem festen Zuschlagswert überschreitet, haben geringe Steigerungen des Momentanwertes kein Deaktivieren des Hochfrequenzgenerators zur Folge, sondern vielmehr einen langsamen Anstieg auch des Effektivwertes. Der Vergleichswert, der zur Detektion von Stromspitzen herangezogen wird, ist somit ebenfalls ein gleitender Vergleichswert und kein fester Wert.

Vorzugsweise ist der von dem Effektivwert des von dem Strom- oder Spannungssensor erfassten Wechselstroms abgeleitete Wert der Effektivwert zuzüglich eines festen Betrages (dem Zuschlagswert). Dieser feste Betrag (oder Zuschlagswert) ist so gewählt, dass gewollte Anstiege des Momentanwertes des Stromes, beispielsweise bei einer gewollten Leistungserhöhung, noch nicht dazu führen, dass die Spitzenwertdetektions- und Steuereinheit den Hochfrequenzgenerator deaktiviert.

Der von dem Effektivwert des von dem Strom- oder Spannungssensor erfassten Wechselstroms abgeleitete Wert kann auch um einen vorgegebenen Faktor (Zuschlagsfaktor), z.B. 1,5 oder 2, größer sein, als der Effektivwert. Es können auch für unterschiedliche Effektivwerte unterschiedliche Zuschlagswerte oder Zuschlagsfaktoren vorgesehen sein.

Alternativ kann das den jeweiligen Momentanwert des Stroms oder der Spannung repräsentierende Signal gedämpft oder abgesenkt sein.

Außerdem ist es bevorzugt, wenn die Spitzenwertdetektions- und Steuereinheit erst mit einer Verzögerung gegenüber einer Aktivierung der Hochspannungs-Stromversorgungseinheit und des Hochfrequenzgenerators aktiviert wird. Unmittelbar nach Starten der Hochspannungs-Stromversorgungseinheit und/oder des Hochfrequenzgenerators steigt nämlich der Momentanwert des von dem Strom- oder Spannungssensor erfassten Stromes von Periode zu Periode relativ stark solange an, bis ein eingeschwungener Zustand erreicht ist. Direkt nach Einschalten der Hochspannungs-Stromversorgungseinheit oder des Hochfrequenzgenerators ist der Effektivwert zunächst null und steigt daraufhin erst langsam an, so dass der Momentanwert regelmäßig deutlich über dem Effektivwert liegt und möglicherweise auch über dem Effektivwert zuzüglich des festen Betrages, also auch über dem von dem Effektivwert abgeleiteten Wert. Damit es in diesem Falle nicht zu einer Deaktivierung des Hochfrequenzgenerators durch die Spitzenwertdetektions- und Steuereinheit kommt, wird diese erst mit einer gewissen Verzögerung aktiviert. Hierzu ist vorzugsweise eine Steuereinheit vorgesehen, die für diese Verzögerung sorgt und entsprechend erst nach einer gewissen Zeitverzögerung ein Aktivierungssignal für die Spitzenwertdetektions- und Steuereinheit zur Verfügung stellt.

Alternativ kann auch vorgesehen sein, dass das den jeweiligen Momentanwert des Stroms oder der Spannung repräsentierende Signal mit einem vorgegebenen Mindestwert verglichen wird und ein Abschaltsignal nur erzeugt wird, wenn das den jeweiligen Momentanwert des Stroms oder der Spannung repräsentierende Signal nicht nur den von dem Effektivwert abgeleiteten Wert, sondern auch den vorgegebenen Mindestwert überschreitet.

Vorzugsweise ist die Spitzenwertdetektions- und Steuereinheit derart mit dem Hochfrequenzgenerator verbunden, dass die Spitzenwertdetektions- und Steuereinheit den Hochfrequenzgenerator durch Unterbinden der Abgabe von Ansteuerungsimpulsen an den Hochfrequenzgenerator deaktiviert. Typischerweise werden einem Hochfrequenzgenerator periodisch Ansteuerungsimpulse mit einer Frequenz zugeführt, die der Resonanzfrequenz des Hochfrequenzgenerators entspricht, um die resonante Schwingung aufrecht zu erhalten. Unterbleibt die Abgabe dieser Ansteuerungsimpulse, gibt der Hochfrequenzgenerator kurzfristig keinen hochfrequenten Wechselstrom mehr ab. Die Spitzenwertdetektions- und Steuereinheit ist vorzugsweise als Hardwarekomponente realisiert, die von dem Strom- oder Spannungssensor stammende analoge Eingangssignale unmittelbar verarbeiten kann. Vorzugsweise enthält die Spitzenwertdetektions- und Steuereinheit einen Komparator zum analogen Vergleich des Momentanwertes des von dem Strom-oder Spannungssensor erfassten Wechselstroms und dem Effektivwert des Wechselstroms. Auf diese Weise kann die Spitzenwertdetektions- und Steuereinheit ein den Hochfrequenzgenerator deaktivierendes Abschaltsignal in kürzestmöglicher Zeit generieren, wenn der Momentanwert des Stroms den von dem Effektivwert abgeleiteten Wert überschreitet.

Vorzugsweise weist die Spitzenwertdetektions- und Steuereinheit außerdem einen Eingang für ein Aktivierungssignal auf, mit dem die Spitzenwertdetektions- und Steuereinheit aktiviert beziehungsweise deaktiviert werden kann. Auf diese Weise ist beispielsweise eine zeitlich verzögerte Aktivierung der Spitzenwertdetektions- und Steuereinheit möglich. Auch kann so vermieden werden, dass es in bestimmten Betriebsmodi zu einem fälschlichen Deaktivieren kommen kann, nämlich dann, wenn sich durch einen entsprechenden Betriebsmodus bedingt Strom- oder Spannungsspitzen ergeben, die gewollt sind. Daher ist es vorteilhaft, wenn die Spitzenwertdetektions- und Steuereinheit bei der Wahl solcher Betriebsmodi deaktiviert wird. Dies kann beispielsweise durch eine softwaregesteuerte zentrale Steuereinheit automatisch immer dann erfolgen, wenn ein Betriebsmodus angewählt oder eingeschaltet wird, der gewollte Strom- oder Spannungsspitzen mit sich bringt.

Gemäß einer weiteren bevorzugten Ausführungsvariante weist die Spitzenwertdetektions-und Steuereinheit einen weiteren Eingang auf, an dem ein Signal anliegt, welches den festen Betrag repräsentiert, durch den sich der von dem Effektivwert abgeleitete Wert von dem Effektivwert unterscheidet. Dieser Eingang für einen festen Betrag repräsentierendes Signal ist vorzugsweise ebenfalls mit einer weiteren Steuereinheit verbunden, beispielsweise mit der Steuereinheit, die auch eine zeitverzögerte Aktivierung der Spitzenwertdetektions- und Steuereinheit bewirkt.

Diese weitere Steuereinheit ist vorzugsweise eine softwaregesteuerte zentrale Steuereinheit, die programmierbar ist und weniger zeitkritische Steuerungsaufgaben übernimmt.

Das Hochfrequenz-Chirurgiegerät weist in einer bevorzugten Ausführungsvariante zusätzlich eine Spannungssteuereinheit auf, die ausgangsseitig mit der Hochspannungs-Stromversorgungseinheit verbunden ist, und die eingangsseitig wenigstens indirekt mit der Spitzenwertdetektions- und Steuereinheit verbunden ist. Diese Spannungssteuereinheit ist ausgebildet, ein Spannungssteuersignal für die Hochspannungs-Stromversorgungseinheit auszugeben. Eingangsseitig ist die Spannungssteuereinheit wenigstens indirekt mit dem Ausgang der Spitzenwertdetektions- und Steuereinheit verbunden, um auf diese Weise das Ausgangssignal an der Spitzenwertdetektions- und Steuereinheit empfangen zu können. Falls die Spitzenwertdetektions- und Steuereinheit ein den Hochfrequenzgenerator deaktivierendes Steuersignal ausgibt, kann dieses Steuersignal gleichzeitig bewirken, dass die Spannungssteuereinheit ein Spannungssteuersignal generiert, das eine Verringerung der von der Hochspannungs-Stromversorgungseinheit abgegebenen Ausgangsspannung bewirkt. Auf diese Weise kann beispielsweise ein sofortiger neuer Spannungsüberschlag vermieden werden, wenn der Hochfrequenzgenerator wieder aktiviert wird.

Die Spannungssteuereinheit ist vorzugsweise als analoge Schaltung mit wenigstens einem weiteren Eingang realisiert, der mit einer Steuereinheit verbunden ist, die softwaregesteuert ist, also beispielsweise mit einer programmierbaren zentralen Steuereinheit des Hochfrequenz-Chirurgiegerätes. Auf diese Weise können die Vorteile einer programmierbaren Steuerung, z.B. für unterschiedliche chirurgische Anwendungen, mit den Vorteilen einer analogen Schaltung - z.B. schnelle Reaktion im Falle eines Lichtbogens - miteinander verbunden werden.

Der Strom-Effektivwertbildner enthält vorzugsweise wenigstens eine Einheit zum Bilden eines quadratischen Mittelwertes (rms).

Die Erfindung soll nun anhand eines Ausführungsbeispiels mit Bezug auf die Figuren näher erläutert werden. Die Figuren zeigen in:
- Fig. 1:: ein schematisches Blockschaltbild eines erfindungsgemäßen Hochfrequenz-Chirurgiegerätes;
- Fig. 2:: ein erstes Diagramm zur Erläuterung der Funktionsweise des Hochfrequenz-Chirurgiegerätes beim Detektieren eines Funkenverschlags; und
- Fig. 3:: ein zweites Diagramm zur Erläuterung der Funktionsweise des Hochfrequenz-Chirurgiegerätes nach dem Einschalten.

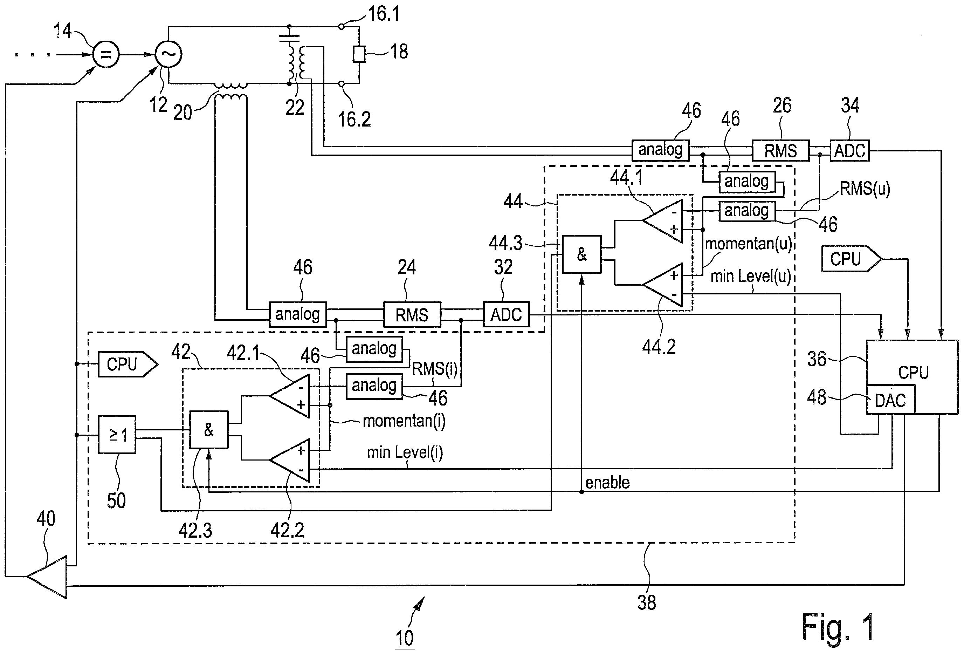

Das in Fig. 1 abgebildete Hochfrequenz-Chirurgiegerät 10 birgt einen Hochfrequenzgenerator 12, der von einer Hochspannungs-Stromversorgungseinheit 14 gespeist wird. Von dem Hochfrequenzgenerator 12 erzeugter hochfrequenter Wechselstrom kann über zwei Anschlüsse 16.1 und 16.2 an ein angeschlossenes Instrument 18 abgegeben werden. Das Instrument 18 stellt für den durch den Hochfrequenzgenerator 12 gespeisten HF-Hochspannungskreis eine Last dar. Der HF-Hochspannungskreis schließt den Hochfrequenzgenerator 12 und das Instrument 18 ein.

Zum Erfassen des im Betrieb in dem HF-Hochspannungskreis fließenden Stroms ist ein Stromsensor 20 vorgesehen, und zum Erfassen der jeweils herrschenden Spannung ist ein Spannungssensor 22 vorgesehen. Die von dem Stromsensor 20 bzw. dem Spannungssensor 22 erfassten Werte werden über analoge Anpassungsschaltungen 46 jeweils einer RMS-Einheit 24 bzw. 26 zugeführt. Die RMS-Einheit 24 bildet einen Effektivwert des Stroms und die RMS-Einheit 26 einen Effektivwert der Spannung. Diese Effektivwerte werden jeweils einem Analog-Digital-Wandler 32 bzw. 34 zugeführt. Die von dem jeweiligen Digital-Wandler 32 bzw. 34 digitalisierten Strom- bzw. Spannungseffektivwerte werden einer softwaregesteuerten zentralen Steuereinheit 36 zugeführt.

Neben der softwaregesteuerten zentralen Steuereinheit 36 sind außerdem zwei in Form von Hardware-Komponenten realisierte, jeweils wenigstens einen Komparator enthaltende Steuereinheiten, nämlich eine Spitzenwertdetektions- und Steuereinheit 38 und eine Spannungssteuereinheit 40, vorgesehen.

Die Spitzenwertdetektions- und Steuereinheit 38 ist ausgangsseitig mit dem Hochfrequenzgenerator 12 verbunden und kann ein Abschaltsignal für den Hochfrequenzgenerator 12 ausgeben.

Die Spitzenwertdetektions- und Steuereinheit 38 weist in dem dargestellten Ausführungsbeispiel eine Spitzenstromdetektionseinheit 42 und eine Spitzenspannungsdetektionseinheit 44 auf. Abweichend hiervon ist es auch möglich, dass die Spitzenwertdetektions- und Steuereinheit 38 nur entweder eine Spitzenstromdetektionseinheit oder eine Spitzenspannungsdetektionseinheit aufweist.

Die Spitzenstromdetektionseinheit 42 und die Spitzenspannungsdetektionseinheit 44 sind grundsätzlich (d.h. abgesehen von Schaltungsdetails, die in der Figur nicht dargestellt sind) gleich aufgebaut. Sowohl die Spitzenstromdetektionseinheit 42 als auch die Spitzenspannungsdetektionseinheit 44 weisen jeweils einen ersten Komparator 42.1 und 44.1 auf, dem jeweils über einen ersten Eingang (in diesem Falle dem jeweiligen invertierenden Eingang) der Effektivwert des Stroms bzw. der Spannung zugeführt wird. Dazu ist der erste (im Beispielsfall invertierende) Eingang des ersten Komparators 42.1 bzw. 44.1 mit einem Ausgang der RMS-Einheit 24 bzw. der RMS-Einheit 26 so verbunden, dass an dem ersten Eingang des ersten Komparators 42.1 bzw. 44.1 ein den jeweiligen Effektivwert des Stroms bzw. der Spannung in dem HF-Hochspannungskreis repräsentierendes Signal anliegt. Ein jeweiliger zweiter (im Ausführungsbeispiel nicht invertierender) Eingang des jeweiligen ersten Komparators 42.1 bzw. 44.1 ist jeweils mit dem Stromsensor 20 bzw. dem Spannungssensor 22 so verbunden, dass an dem zweiten Eingang ein den jeweiligen Momentanwert des Stroms bzw. der Spannung repräsentierendes Signal anliegt. Damit wird dem jeweiligen zweiten Eingang des jeweiligen ersten Komparators 42.1 bzw. 44.1 im Betrieb jeweils ein den Momentanwert des Stroms bzw. der Spannung repräsentierendes Signal zugeführt, sodass der jeweilige erste Komparator 42.1 bzw. 44.1 einen jeweiligen Momentanwert des Stroms bzw. der Spannung mit einem von dem entsprechenden Effektivwert abgeleiteten Wert des Stroms bzw. der Spannung vergleicht. Den beiden Eingängen sind jeweils analoge Anpassungsschaltungen 46 vorgeschaltet, die nötigenfalls für eine Strom- oder Spannungswandlung bzw. eine Impedanzanpassung sorgen.

Mittels der analogen Anpassungsschaltungen kann der jeweilige Effektivwert in Bezug auf die ihm zugrundeliegenden Momentanwerte erhöht werden und so zu einem von dem Strom- oder Spannungssensor im Betrieb erfassten hochfrequenten Wechselstrom abgeleiteten Wert werden.

Eine derartige relative Vergrößerung des jeweiligen Effektivwertes gegenüber den ihm zugrundeliegenden Momentanwerten kann auch durch Dämpfen (d.h. Verringern der Amplitude) der Momentanwerte mittels entsprechender Anpassungsschaltungen 46 erfolgen.

Der Ausgang des jeweiligen ersten Komparators 42.1 bzw. 44.1 liefert den Spannungswert, der einer logischen 1 entspricht, wenn der Momentanwert des Stroms bzw. der Spannung den von dem entsprechenden Effektivwert abgeleiteten Wert übersteigt.

Die Spitzenstromdetektionseinheit 42 und die Spitzenspannungsdetektionseinheit 44 weisen darüber hinaus jeweils noch einen zweiten Komparator 42.2 und 44.2 auf, deren jeweiliger nicht invertierender Eingang genau wie der entsprechende Eingang des jeweiligen ersten Komparators 42.1 bzw. 44.1 jeweils mit dem Stromsensor 20 bzw. dem Spannungssensor 22 so verbunden ist, dass an dem nicht invertierenden Eingang des jeweiligen zweiten Komparators 42.2 bzw. 44.2 ebenfalls ein einen jeweiligen Momentanwert des Stroms bzw. der Spannung repräsentierendes Signal anliegt. Damit wird auch dem jeweiligen nicht invertierenden Eingang des jeweiligen zweiten Komparators 42.2 bzw. 44.2 im Betrieb jeweils ein dem Momentanwert des Stroms bzw. der Spannung repräsentierendes Signal zugeführt.

Der jeweilige invertierende Eingang des jeweiligen zweiten Komparators 42.2 bzw. 44.2 ist über einen Digital-Analog-Wandler 48 mit der softwaregesteuerten zentralen Steuereinheit 36 verbunden. Die softwaregesteuerte zentrale Steuereinheit 36 kann auf diese Weise einen Mindestwert für den Momentanwert des Stroms bzw. der Spannung vorgeben, der überschritten werden muss, damit der jeweilige zweite Komparator 42.2 bzw. 44.2 ein einer logischen 1 entsprechendes Ausgangssignal liefert.

Die beiden Ausgangssignale des jeweiligen ersten Komparators 42.1 bzw. 44.1 und des jeweiligen zweiten Komparators 42.2 bzw. 44.2 werden jeweils einem Und-Glied 42.3 bzw. 44.3 zugeführt. Das jeweilige Und-Glied 42.3 bzw. 44.3 liefert an seinem Ausgang nur dann eine logische 1 repräsentierendes Signal, wenn beide Komparatoren der Spitzenstromdetektionseinheit 42 bzw. der Spitzenspannungsdetektionseinheit 44 jeweils eine logische 1 repräsentierendes Ausgangssignal liefern. Dies ist dann der Fall, wenn der jeweilige Momentanwert des Stroms bzw. der Spannung sowohl einen jeweiligen von der softwaregesteuerten zentralen Steuereinheit 36 vorgegebenen Mindestwert des Stroms bzw. der Spannung überschreitet als auch den jeweiligen Effektivwert des Stroms oder der Spannung. Im Ergebnis bedeutet dies, dass sowohl die Spitzenstromdetektionseinheit 42 als auch die Spitzenspannungsdetektionseinheit 44 nur dann ein einer logischen 1 entsprechendes Ausgangssignal liefern, wenn der den Hochfrequenzgenerator 12 und das Instrument 18 einschließende HF-Hochspannungskreis soweit eingeschwungen ist, dass der Momentanwert des Stroms oder der Spannung in jedem Falle den von der softwaregesteuerten zentralen Steuereinheit 36 vorgegebenen Mindestwert überschreiten.

Die beiden Und-Glieder 42.3 bzw. 44.3 weisen jeweils einen Aktivierungseingang auf, über den die Und-Glieder 42.3 bzw. 44.3 von der softwaregesteuerten zentralen Steuereinheit 36 aktiviert werden können. Der jeweilige Aktivierungseingang der Und-Glieder 42.3 und 44.3 ist somit ebenfalls mit der softwaregesteuerten zentralen Steuereinheit 36 verbunden und ausgebildet, von dieser ein Enable-Signal zu empfangen, um dadurch aktiviert zu werden. Bleibt ein derartiges Enable-Signal aus, ist die Spitzenwertdetektions-und Steuereinheit 38 nicht aktiviert.

Die Ausgangssignale der Spitzenstromdetektionseinheit 42 und der Spitzenspannungsdetektionseinheit 44 (also jeweils eine logische "1" oder logische "0" repräsentierende Signale) werden einem Oder-Glied 50 der Spitzenwertdetektions- und Steuereinheit 38 zugeführt, sodass die Spitzenwertdetektions- und Steuereinheit 38 immer dann ein eine logische 1 repräsentierendes Ausgangssignal liefert, wenn die beiden Und-Glieder 42.3 und 44.3 jeweils durch ein entsprechendes Enable-Signal der softwaregesteuerten zentralen Steuereinheit 36 aktiviert sind und außerdem der Momentanwert des Stroms oder der Momentanwert der Spannung sowohl den jeweils durch die softwaregesteuerte zentrale Steuereinheit 36 vorgegebenen jeweiligen Mindestwert überschreitet als auch den jeweiligen Effektivwert des Stroms oder der Spannung. Das Ausgangssignal der Spitzenwertdetektions- und Steuereinheit 38 wird einerseits der softwaregesteuerten zentralen Steuereinheit 36 zugeführt und andererseits als Abschaltsignal dem Hochfrequenzgenerator 12. Dies bedeutet, dass der Hochfrequenzgenerator 12 keine Ansteuerungsimpulse mehr erhält, wenn der Ausgang der Spitzenwertdetektions- und Steuereinheit 38 ein eine logische 1 repräsentierendes Signal liefert. Damit kann der Hochfrequenzgenerator 12 in kürzester Zeit deaktiviert werden.

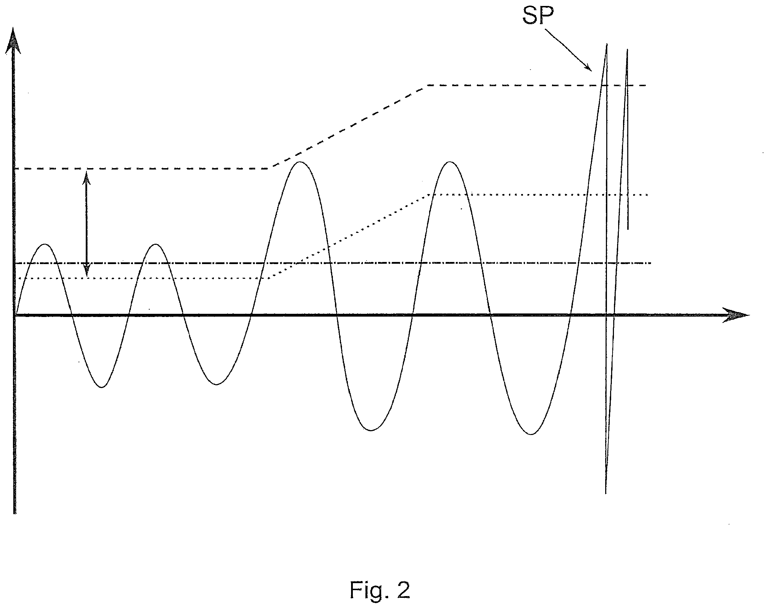

Wie in Fig. 2 noch näher illustriert, erzeugt die Spitzenwertdetektions- und Steuereinheit 38 somit dann ein Abschaltsignal und gibt dieses an den Hochfrequenzgenerator 12 aus, wenn ein Momentanwert des den Effektivwert des Stroms sowie den jeweiligen von der softwaregesteuerten zentralen Steuereinheit 36 vorgegebenen Mindestwert überschreitet. Das Abschaltsignal bewirkt, dass eine Abgabe von Ansteuerungsimpulsen an den Hochfrequenzgenerator 12 unterbleibt, so dass dieser im Ergebnis keinen hochfrequenten Wechselstrom mehr abgibt.

Figur 2 zeigt in einer durchgezogenen Linie den Momentanwert des (Wechsel-) Stroms oder der (Wechsel-) Spannung in dem HF-Hochspannungskreis. Ein diesen Momentanwert repräsentierendes Signal liegt an den nicht-invertierenden Eingängen der Komparatoren 42.1, 44.1, 42.2 und 44.2 an. Eine strichpunktierte Linie repräsentiert den von der softwaregesteuerten zentralen Steuereinheit 36 vorgegebenen Mindestwert, der am jeweiligen invertierenden Eingang des entsprechenden zweiten Komparators 42.4 bzw. 44.2 anliegt. Eine punktierte Line repräsentiert den Effektivwert des Stroms oder der Spannung in dem HF-Hochspannungskreis. Eine gestrichelte Line deutet den um einen festen Betrag erhöhten Effektivwert des Stroms oder der Spannung in dem HF-Hochspannungskreis, und damit einen von dem Strom- oder Spannungssensor im Betrieb erfassten hochfrequenten Wechselstrom abgeleiteten Wert, so wie er dem invertierenden Eingang des entsprechenden ersten Komparators 42.1 bzw. 44.1 zugeführt wird.

Da der vom Effektivwert abgeleitete Wert so gewählt ist, dass der Momentanwert bei einen gewollten Veränderung der abgegebenen Leistung den vom Effektivwert abgeleiteten Wert noch nicht überschreitet, ein Leistungsanstieg möglich, wie Figur 2 zeigt. Erst wenn es zu einem plötzlichen und starken Anstieg wie z.B. im Falle eines Funkenüberschlags kommt, überschreitet der Momentanwert den vom Effektivwert abgeleiteten Wert und die Spitzenwertdetektions- und Steuereinheit 38 spricht an; siehe Figur 2 bei "SP".

Da der Vergleich des jeweiligen Momentanwertes des Stroms mit dem Effektivwert des Stroms mittels eines Komparators der Spitzenwertdetektions- und Steuereinheit 38 erfolgt, wird das Abschaltsignal gegebenenfalls sehr schnell erzeugt und ein Abschalten der Ansteuerungsimpulse für den Hochfrequenzgenerator 12 kann innerhalb weniger µs, beispielsweise innerhalb von 5 µs, nach Auftreten einer Stromspitze beispielsweise infolge eines Funkenüberschlags, erfolgen.

Das Abschaltsignal wird nicht nur dem Hochfrequenzgenerator 12 zugeführt, sondern außerdem der Spannungssteuereinheit 40, die ebenfalls als Hardwaresteuerung ausgeführt ist. Die Spannungssteuereinheit 40 erzeugt als Ausgangssignal ein Steuersignal für die Hochspannungs-Stromversorgungseinheit 14. Eingangswerte der Spannungssteuereinheit 40 sind neben dem Abschaltsignal von der Spitzenwertdetektions- und Steuereinheit 38 auch ein von der zentralen Steuereinheit 36 generierter Referenzwert. Da die zentrale Steuereinheit softwaregesteuert ist, wird der von der zentralen Steuereinheit 36 erzeugte Referenzwert mittels jeweils des Digital-Analog-Wandlers 48 in einen analogen Referenzwert gewandelt.

Indem der Spannungssteuereinheit 40 auch das Abschaltsignal der Spitzenwertdetektions- und Steuereinheit 38 zugeführt wird, kann die Spannungssteuereinheit 40 im Falle eines Funkenüberschlags ein Steuersignal erzeugen, das zu einer Verringerung der Ausgangsspannung der Hochspannungs-Stromversorgungseinheit 14 führt. Da dies auf rein analogem Wege erfolgt, erfolgt die Spannungsregelung im Falle eines Funkenüberschlags ebenfalls sehr schnell. Ansonsten erfolgt die Spannungsregelung über die softwaregesteuerte zentrale Steuereinheit 36 und die von dieser generierten Strom- bzw. Spannungswerte.

Die Spannungssteuereinheit 40 kann als Und-Glied oder als Oder-Glied oder auch als Monoflop ausgebildet sein.

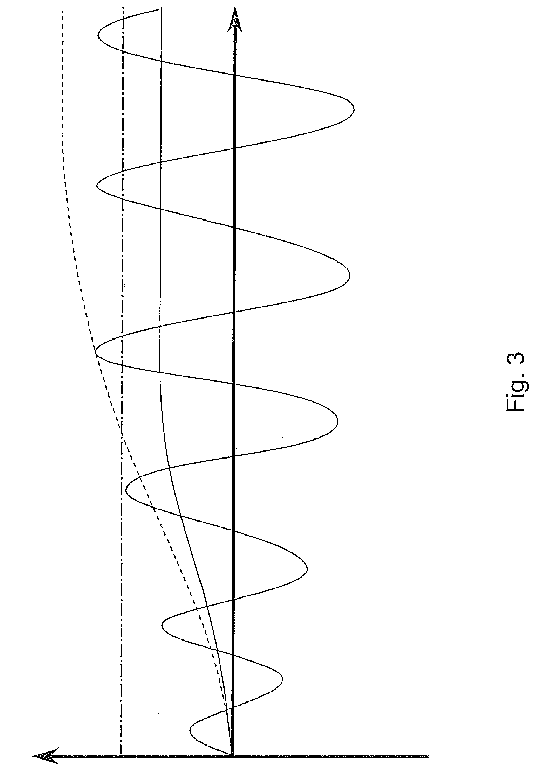

Figur 3 zeigt, dass die Spitzenwertdetektions- und Steuereinheit 38 dann anspricht, wenn der jeweilige Momentanwert sowohl den vom Effektivwert abgeleiteten Wert (gestrichelte Linie) als auch den vorgegeben Mindestwert (strichpunktierte Line) überschreitet. Damit wird verhindert, dass die Spitzenwertdetektions- und Steuereinheit 38 nicht schon dann anspricht, wenn der Momentanwert den vom Effektivwert abgeleiteten Wert unmittelbar nach dem Einschalten des Hochfrequenzgenerator 12 allein deshalb überschreitet, weil sich der HF-Hochspannungskreis gerade erst einschwingt und der Effektivwert erst langsam der beim Einschwingen schnell ansteigenden Maximal-Amplitude des Momentanwertes folgt.

### Bezugszeichenliste

- 10: Hochfrequenz-Chirurgiegerät
- 12: Hochfrequenzgenerator
- 14: Hochspannungs-Stromversorgungseinheit
- 16.1 und 16.2: Anschlüsse
- 18: Instrument
- 20: Stromsensor
- 22: Spannungssensor
- 24: Strom-RMS-Einheit.
- 26: Spannungs-RMS-Einheit
- 32, 34: Analog-Digital-Wandler
- 36: zentrale Steuereinheit
- 38: Spitzenwertdetektions- und Steuereinheit
- 40: Spannungssteuereinheit
- 42: Spitzenstromdetektions- und Steuereinheit
- 44: Spitzenspannungsdetektions- und Steuereinheit
- 42.1, 42.2, 44.1, 44.2: Komparator
- 42.3, 44.3: Und-Glied
- 46: Anpassungsschaltung
- 48: Digital-Analog-Wandler
- 50: Oder-Glied

## Patentansprüche

1. Hochfrequenz-Chirurgiegerät (10) mit einer Hochspannungs-Stromversorgungseinheit (14) und einem Hochfrequenzgenerator (12), der von der Hochspannungs-Stromversorgungseinheit (14) energieversorgt wird und im Betrieb einen hochfrequenten Wechselstrom erzeugt und an eine Last abgibt, sowie mit einer Spitzenwertdetektions- und Steuereinheit (38), die einen Strom- oder Spannungssensor (20, 22), der zum Erfassen der Stromstärke oder der Spannung eines im Betrieb an die Last abgegebenen hochfrequenten Wechselstroms ausgebildet und angeordnet ist, und einen Effektivwertbildner aufweist oder mit diesen verbunden ist, der mit dem Strom- oder Spannungssensor (20, 22) verbunden und ausgebildet ist, einen Effektivwert eines von dem Strom-oder Spannungssensor (20, 22) erfassten hochfrequenten Wechselstroms zu bilden, wobei die Spitzenwertdetektions- und Steuereinheit (38) ausgebildet ist dann, wenn ein Betrag eines einen Momentanwert des von dem Strom- oder Spannungssensor (20, 22) im Betrieb erfassten hochfrequenten Wechselstroms repräsentierenden Signals einen Betrag eines von dem Effektivwert des von dem Strom- oder Spannungssensor (20, 22) im Betrieb erfassten hochfrequenten Wechselstroms abgeleiteten Wertes überschreitet, den Hochfrequenzgenerator (12) zu deaktivieren, **dadurch gekennzeichnet, dass** der von dem Effektivwert des von dem Strom- oder Spannungssensor (20, 22) im Betrieb erfassten hochfrequenten Wechselstroms abgeleitete Wert dem um ein vorgegebenes Maß hinsichtlich des Betrags erhöhten Absolutwert des Effektivwerts oder dem Effektivwert entspricht und die Maximalamplitude des repräsentierenden Signals im stabilen, eingeschwungenen Zustand des HF-Hochspannungskreises kleiner ist als der abgeleitete Wert.

2. Hochfrequenz-Chirurgiegerät (10) nach Anspruch 1, **dadurch gekennzeichnet, dass** der von dem Effektivwert des von dem Strom- oder Spannungssensor (20, 22) im Betrieb erfassten hochfrequenten Wechselstroms abgeleitete Wert dem um einen Faktor erhöhten Absolutwert des Effektivwerts oder dem Absolutwert des Effektivwerts zuzüglich eines vorgegebenen Betrages entspricht.

3. Hochfrequenz-Chirurgiegerät (10) nach wenigstens einem der Ansprüche 1 bis 2, **dadurch gekennzeichnet, dass** das einen Momentanwert des von dem Strom- oder Spannungssensor (20, 22) im Betrieb erfassten hochfrequenten Wechselstroms repräsentierende Signal gedämpft oder hinsichtlich seines Betrags verringert ist.

4. Hochfrequenz-Chirurgiegerät (10) nach wenigstens einem der Ansprüche 1 bis 3, **dadurch gekennzeichnet, dass** der Hochfrequenzgenerator (12) und die Spitzenwertdetektions- und Steuereinheit (38) so miteinander verbunden sind, dass die Spitzenwertdetektions- und Steuereinheit (38) den Hochfrequenzgenerator (12) durch Unterbinden der Abgabe von Ansteuerungsimpulsen an den Hochfrequenzgenerator (12) deaktiviert.

5. Hochfrequenz-Chirurgiegerät (10) nach wenigstens einem der Ansprüche 1 bis 4, **dadurch gekennzeichnet, dass** die Spitzenwertdetektions- und Steuereinheit (38) als analoge Schaltung realisiert ist, die vom Stromsensor (20) stammende analoge Signale analog verarbeitet und dazu einen Komparator (42.1, 42.2, 44.1, 44.2) enthält.

6. Hochfrequenz-Chirurgiegerät (10) nach wenigstens einem der Ansprüche 1 bis 5, **dadurch gekennzeichnet, dass** die Spitzenwertdetektions- und Steuereinheit (38) derart ausgebildet und mit dem Hochfrequenzgenerator (12) verbunden ist, dass die Spitzenwertdetektions- und Steuereinheit (38) den Hochfrequenzgenerator (12) durch Unterbinden der Abgabe von Ansteuerungsimpulsen an den Hochfrequenzgenerator (12) deaktiviert.

7. Hochfrequenz-Chirurgiegerät (10) nach wenigstens einem der Ansprüche 1 bis 6, **dadurch gekennzeichnet, dass** die Spitzenwertdetektions- und Steuereinheit (38) einen Eingang für ein Aktivierungssignal aufweist.

8. Hochfrequenz-Chirurgiegerät (10) nach wenigstens einem der Ansprüche 1 bis 7, mit einer Steuereinheit, **dadurch gekennzeichnet, dass** die Steuereinheit ausgebildet ist, die Spitzenwertdetektions- und Steuereinheit (38) mit einer Verzögerung gegenüber einer Aktivierung der Hochspannungs-Stromversorgungseinheit (14) oder des Hochfrequenzgenerators (12) zu aktivieren.

9. Hochfrequenz-Chirurgiegerät (10) nach wenigstens einem der Ansprüche 1 bis 8, **dadurch gekennzeichnet, dass** die Spitzenwertdetektions- und Steuereinheit (38) einen Eingang aufweist, an dem ein Signal anliegt, welches den festen Betrag oder den Faktor repräsentiert, durch den sich der von dem Absolutwert des Effektivwerts abgeleitete Wert von dem Absolutwert des Effektivwerts unterscheidet.

10. Hochfrequenz-Chirurgiegerät (10) nach Anspruch 9, **dadurch gekennzeichnet, dass** der Eingang für das den festen Betrag oder den Faktor repräsentierende Signal mit einer Steuereinheit verbunden ist, die softwaregesteuert ist.

11. Hochfrequenz-Chirurgiegerät (10) nach wenigstens einem der Ansprüche 1 bis 10, **gekennzeichnet durch** eine Spanungssteuereinheit, die ausgangsseitig mit der Hochspannungs-Stromversorgungseinheit (14) verbunden ist und die eingangsseitig wenigstens indirekt mit der Spitzenwertdetektions- und Steuereinheit (38) verbunden ist, und die ausgebildet ist, auf ein Ausgangssignal der Spitzenwertdetektions- und Steuereinheit (38) hin ein Spannungssteuersignal als Ausgangssignal zu erzeugen, dass eine Verringerung der von der Hochspannungs-Stromversorgungseinheit (14) abgegebenen Spannung bewirkt.

12. Hochfrequenz-Chirurgiegerät (10) nach Anspruch 11, **dadurch gekennzeichnet, dass** die Spannungssteuereinheit (40) als analoge Schaltung mit wenigstens einem weiteren Eingang realisiert ist, der mit einer Steuereinheit verbunden ist, die softwaregesteuert ist.

13. Hochfrequenz-Chirurgiegerät (10) nach wenigstens einem der Ansprüche 1 bis 12, **dadurch gekennzeichnet, dass** der Strom-Effektivwertbildner wenigstens eine Einheit zum Bilden eines quadratischen Mittelwertes (rms) enthält.

## Claims

1. A high-frequency surgical appliance (10) having a high-voltage power supply unit (14) and a high-frequency generator (12), which is supplied with energy by the high-voltage power supply unit (14) and, during operation, generates a high-frequency alternating current and delivers it to a load, and having a peak value detection and control unit (38) which comprises or is connected to a current or voltage sensor (20, 22), which is configured and arranged in order to detect the current strength or the voltage of a high-frequency alternating current delivered to the load during operation, and an rms value forming unit, which is connected to the current or voltage sensor (20, 22) and is configured in order to form an rms value of a high-frequency alternating current detected by the current or voltage sensor (20, 22), wherein
the peak value detection and control unit (38) is configured in order to deactivate the high-frequency generator (12) whenever a magnitude of a signal representing an instantaneous value of the high-frequency alternating current detected by the current or voltage sensor (20, 22) during operation exceeds a magnitude of a value derived from the rms value of the high-frequency alternating current detected by the current or voltage sensor (20, 22) during operation,
**characterized in that** the value derived from the rms value of the high-frequency alternating current detected by the current or voltage sensor (20, 22) during operation corresponds to the absolute value, increased by a predetermined extent in respect of its magnitude, of the rms value or to the rms value and the maximum amplitude of the representing signal in the stable settled state of the HF high-voltage circuit is less than the derived value.

2. The high-frequency surgical appliance (10) as claimed in claim 1, **characterized in that** the value derived from the rms value of the high-frequency alternating current detected by the current or voltage sensor (20, 22) during operation corresponds to the absolute value, increased by a factor, of the rms value or to the absolute value of the rms value plus a predetermined magnitude.

3. The high-frequency surgical appliance (10) as claimed in at least one of claims 1 to 2, **characterized in that** the signal representing an instantaneous value of the high-frequency alternating current detected by the current or voltage sensor (20, 22) during operation is attenuated or is reduced in respect of its magnitude.

4. The high-frequency surgical appliance (10) as claimed in at least one of claims 1 to 3, **characterized in that** the high-frequency generator (12) and the peak value detection and control unit (38) are connected to one another in such a way that the peak value detection and control unit (38) deactivates the high-frequency generator (12) by preventing the delivery of drive pulses to the high-frequency generator (12).

5. The high-frequency surgical appliance (10) as claimed in at least one of claims 1 to 4, **characterized in that** the peak value detection and control unit (38) is produced as an analog circuit, which processes analog signals coming from the current sensor (20) in an analog fashion and to this end contains a comparator (42.1, 42.2, 44.1, 44.2).

6. The high-frequency surgical appliance (10) as claimed in at least one of claims 1 to 5, **characterized in that** the peak value detection and control unit (38) is configured, and connected to the high-frequency generator (12), in such a way that the peak value detection and control unit (38) deactivates the high-frequency generator (12) by preventing the delivery of drive pulses to the high-frequency generator (12).

7. The high-frequency surgical appliance (10) as claimed in at least one of claims 1 to 6, **characterized in that** the peak value detection and control unit (38) has an input for an activation signal.

8. The high-frequency surgical appliance (10) as claimed in at least one of claims 1 to 7, having a control unity **characterized in that** the control unit is configured in order to activate the peak value detection and control unit (38) with a time delay relative to an activation of the high-voltage power supply unit (14) or of the high-frequency generator (12).

9. The high-frequency surgical appliance (10) as claimed in at least one of claims 1 to 8, **characterized in that** the peak value detection and control unit (38) has an input to which a signal is applied that represents the fixed magnitude or the factor by which the value derived from the absolute value of the rms value differs from the absolute value of the rms value.

10. The high-frequency surgical appliance (10) as claimed in claim 9, **characterized in that** the input for the signal representing the fixed magnitude or the factor is connected to a control unit which is software-controlled.

11. The high-frequency surgical appliance (10) as claimed in at least one of claims 1 to 10, **characterized by** a voltage control unit which is connected on the output side to the high-voltage power supply unit (14) and which is connected on the input side at least indirectly to the peak value detection and control unit (38), and which is configured in order to generate as an output signal, in response to an output signal of the peak value detection and control unit (38), a voltage control signal which causes a reduction of the voltage delivered by the high-voltage power supply unit (14).

12. The high-frequency surgical appliance (10) as claimed in claim 11, **characterized in that** the voltage control unit (40) is produced as an analog circuit having at least one further input, which is connected to a control unit that is software-controlled.

13. The high-frequency surgical appliance (10) as claimed in at least one of claims 1 to 12, **characterized in that** the current rms value forming unit contains at least one unit for forming a root mean square value (rms).

## Revendications

1. Appareil (10) chirurgical à haute fréquence comprenant une unité (14) d'alimentation en courant à haute tension et un générateur (12) à haute fréquence, qui est alimenté en énergie par l'unité (14) d'alimentation en courant à haute tension et qui produit en fonctionnement un courant alternatif à haute fréquence et le donne à une charge, ainsi qu'une unité (38) de détection de valeur de pointe et de commande, qui a un capteur (20, 22) de courant ou de tension, constitué et disposé pour relever l'intensité ou la tension d'un courant alternatif à haute fréquence donné à la charge en fonctionnement, et un formeur de valeur efficace ou est relié à celui-ci, qui est relié au capteur (20, 22) de courant et de tension et est constitué pour former une valeur efficace d'un courant alternatif à haute fréquence relevé par le capteur (20, 22) de courant ou de tension,
dans lequel l'unité (38) de détection de valeur de pointe et de commande est constituée pour, si un montant d'un signal, représentant une valeur instantanée du courant alternatif à haute fréquence relevé en fonctionnement par le capteur (20, 22) de courant ou de tension, dépasse un montant d'une valeur déduite de la valeur efficace du courant alternatif à haute fréquence relevé en fonctionnement par le capteur (20, 22) de courant ou de tension, désactiver le générateur (12) de haute fréquence,
**caractérisé en ce que**
la valeur déduite de la valeur efficace du courant alternatif à haute fréquence relevé en fonctionnement par le capteur (20, 22) de courant ou de tension correspond à la valeur absolue, augmentée d'une mesure donnée à l'avance en ce qui concerne le montant, de la valeur efficace ou à la valeur efficace et l'amplitude maximum du signal représentant, à l'état stable amorcé du circuit à haute tension HF, est plus petite que la valeur déduite.

2. Appareil (10) chirurgical à haute fréquence suivant la revendication 1, **caractérisé en ce que**
la valeur déduite de la valeur efficace du courant alternatif à haute fréquence relevé en fonctionnement par le capteur (20, 22) de courant ou de tension correspond à la valeur absolue, augmentée d'un facteur, de la valeur efficace ou à la valeur absolue de la valeur efficace additionnée d'un montant donné à l'avance.

3. Appareil (10) chirurgical à haute fréquence suivant au moins l'une des revendications 1 à 2, **caractérisé en ce que**
le signal représentant une valeur instantanée du courant alternatif à haute fréquence relevé en fonctionnement par le capteur (20, 22) de courant ou de tension est amorti ou est diminué en ce qui concerne son montant.

4. Appareil (10) chirurgical à haute fréquence suivant au moins l'une des revendications 1 à 3, **caractérisé en ce que** le générateur (12) à haute fréquence et l'unité (38) de détection de valeur de pointe et de commande sont reliés l'un à l'autre, de manière à ce que l'unité (38) de détection de valeur de pointe et de commande désactive le générateur (12) à haute fréquence par suppression de l'envoi d'impulsions de commande au générateur (12) à haute fréquence.

5. Appareil (10) chirurgical à haute fréquence suivant au moins l'une des revendications 1 à 4, **caractérisé en ce que** l'unité de détection de pointe et de commande est réalisée en circuit analogique, qui traite analogiquement des signaux analogiques provenant du capteur (20) de courant et contient à cet effet un comparateur (42.1, 42.2, 44.1, 44.2).

6. Appareil (10) chirurgical à haute fréquence suivant au moins l'une des revendications 1 à 5, **caractérisé en ce que** l'unité (38) de détection de valeur de pointe et de commande est constituée et est reliée au générateur (12) à haute fréquence de manière à ce que l'unité (38) de détection de valeur de pointe et de commande désactive le générateur (12) à haute fréquence en supprimant l'envoi d'impulsions de commande au générateur (12) à haute fréquence.

7. Appareil (10) chirurgical à haute fréquence suivant au moins l'une des revendications 1 à 6, **caractérisé en ce que** l'unité (38) de détection de valeur de pointe et de commande a une entrée d'un signal d'activation.

8. Appareil (10) chirurgical à haute fréquence suivant au moins l'une des revendications 1 à 7, comprenant une unité de commande, **caractérisé en ce que** l'unité de commande est constituée pour activer l'unité (38) de détection de valeur de pointe et de commande avec un retard par rapport à une activation de l'unité (14) d'alimentation en courant - en haute tension ou du générateur (12) à haute fréquence.

9. Appareil (10) chirurgical à haute fréquence suivant au moins l'une des revendications 1 à 8, **caractérisé en ce que** l'unité (38) de détection de valeur de pointe et de commande a une entrée à laquelle s'applique un signal, qui représente le montant fixe ou le facteur par lequel la valeur déduite de la valeur absolue de la valeur efficace se différencie de la valeur absolue de la valeur efficace.

10. Appareil (10) chirurgical à haute fréquence suivant la revendication 9, **caractérisé en ce que** l'entrée du signal représentant le montant fixe ou le facteur est reliée à une unité de commande, qui est commandée en logiciel.

11. Appareil (10) chirurgical à haute fréquence suivant au moins l'une des revendications 1 à 10, **caractérisé par** une unité de commande de la tension, qui est reliée du côté de la sortie à l'unité (14) d'alimentation en courant - haute tension et qui est reliée du côté de l'entrée au moins indirectement à l'unité (38) de détection de valeur de pointe et de commande et qui est constituée pour produire, sur un signal de sortie de l'unité (38) de détection de valeur de pointe et de commande, un signal de commande de tension comme signal de sortie, qui provoque une diminution de la tension appliquée par l'unité (14) d'alimentation en courant - haute tension.

12. Appareil (10) chirurgical à haute fréquence suivant la revendication 11, **caractérisé en ce que** l'unité (40) de commande de la tension est réalisée sous la forme d'un circuit analogique ayant au moins une autre entrée, qui est reliée à une unité de commande commandée en logiciel.

13. Appareil (10) chirurgical à haute fréquence suivant au moins l'une des revendications 1 à 12, **caractérisé en ce que** le formeur de valeur efficace de courant comporte au moins une unité de formation d'une valeur moyenne quadratique (rms).
